# EUROPEAN PATENT SPECIFICATION

(11) **EP 3 634 362 B1**
(45) Date of publication and mention of the grant of the patent: **23.12.2020**
(21) Application number: 18727005.3
(22) Date of filing: 25.05.2018
(51) Int. Cl.: A61K 8/24, A61K 8/25, A61K 8/19, A61Q 11/00, A61K 31/198

(54) **ORAL CARE COMPOSITION**
MUNDPFLEGEZUSAMMENSETZUNG
COMPOSITION D'HYGIÈNE BUCCALE

(30) Priority: 07.06.2017 WO PCT/CN2017/087421; 07.07.2017 EP 17180180
(43) Date of publication of application: 15.04.2020
(73) Proprietor: Unilever N.V., 3013 AL Rotterdam (NL); Unilever PLC, London Greater London EC4Y 0DY (GB)
(72) Inventor: LI, Xiaoke, Shanghai 200335 (CN); TANG, Ruikang, Hangzhou Zhejiang 310058 (CN); WANG, Jinfang, Shanghai 200335 (CN); WANG, Yanan, Hangzhou Zhejiang 310058 (CN); XU, Xurong, Hangzhou Zhejiang 310058 (CN); ZHANG, Meili, Shanghai 200335 (CN)
(74) Representative: Tansley, Sally Elizabeth
(86) International application number: PCT/EP2018/063753
(87) International publication number: WO 2018/224331

(56) References cited:
- WO-A1-2014/147630
- WO-A2-2012/031785
- CA-A1- 2 875 084
- US-A1- 2012 141 588

## Description

### Technical Field of the Invention

The present invention relates to oral care compositions such as tooth pastes, gums, mouthwashes and the like. In particular the present invention relates to an oral care composition comprising a calcium source, a water soluble phosphate source, a water soluble silicate source and an amino acid. The invention also relates to the use of such compositions for remineralisation and/or whitening of teeth of an individual.

### Background of the Invention

Many products we consume have a negative impact on our teeth and mouth. Acidic drinks and sweets, for example, can result in tooth erosion by attacking enamel which is the outer coating that protects the teeth. Moreover, tobacco based products as well as beverages like coffee and tea can stain or reduce the whiteness of one's teeth. These staining and discolouring substances are often able to permeate the enamel layer. This problem occurs gradually over many years, but imparts a noticeable discoloration of the enamel of one's teeth.

In addition to what we consume, the natural equilibrium between tooth hydroxyapatite being dissolved from the enamel of teeth and hydroxyapatite being formed on or in teeth from substances occurring naturally in the saliva shifts continuously. Such a shift can yield unattractive teeth with cariogenic conditions. A variety of products are currently used for addressing tooth decay and/or tooth whitening. Such products often comprise peroxides, abrasives or both. These types of products are often not desired since they do not contribute to the remineralisation of teeth and can cause damage to teeth and gums if overused. Products comprising calcium source have been developed in an attempt to enhance tooth remineralisation. Such products may generate a new remineralisation layer that bind to tooth surfaces.

There is continuing need for improving tooth remineralisation efficacy such as enhancing the adhesion of a new remineralisation layer to tooth surfaces to prevent it being rinsed out of the mouth in wash water during one's daily oral hygiene routine. The present inventors have now found unexpectedly that an oral care composition comprising a calcium source, a water soluble phosphate source, a water soluble silicate source and an amino acid can induce the formation of smaller hydroxyapatite (HAP) crystallites to form a dense remineralisation layer on tooth surfaces, which result in an enhancement of the binding affinity of the new remineralisation layer to tooth surfaces.

As the outer coating of the teeth, enamel is composed of numerous needle-like apatite crystallites, which are bundled in parallel ordered prisms to ensure the unique mechanical strength and biological protection. It has always been of great interest for people to build ordered structures during tooth remineralisation which resemble native enamel structure. Various attempts to remineralise teeth have been extensively studied, but HAP crystallites formed in a remineralisation layer are relatively disordered compared to those in native enamel, and the remineralisation layer is far from enamel-like structure.

The present inventors have further found unexpectedly that an oral care composition of the present invention comprising additional polyacrylic acid (PAA) can even regulate the aggregation and crystallisation of HAP crystallites formed on tooth surfaces into ordered structures which have the morphology and orientation similar to those of native enamel.

### Additional Information

WO 2011/110414 A (Unilever) discloses a single-phase oral care composition comprising both calcium and phosphate sources and is substantially free of water. The single-phase oral care composition is stable, maintains good viscosity characteristics and avoids the need for compartmentalised packaging. The calcium source is a water insoluble and/or slightly soluble calcium source.

The reference above does not describe an oral care composition comprising a calcium source, a water soluble phosphate source, a water soluble silicate source, an amino acid and particularly such an oral care composition can form a dense remineralisation layer that binds with high affinity to tooth surfaces.

### Tests and Definitions

### Dentifrice

"Dentifrice" for the purposes of the present invention means a paste, powder, liquid, gum or other preparation for cleaning the teeth or other surfaces in the oral cavity.

### Tooth Paste

"Tooth paste" for the purpose of the present invention means a paste or gel dentifrice for use with a toothbrush. Especially preferred are tooth pastes suitable for cleaning teeth by brushing for about two minutes.

### Mouth Wash

"Mouth wash" for the purpose of the present invention means liquid dentifrice for use in rinsing the mouth. Especially preferred are mouth washes suitable for rinsing the mouth by swishing and/or gargling for about half a minute before expectorating.

### pH

pH is quoted at atmospheric pressure and a temperature of 25°C. When referring to the pH of an oral care composition, this means the pH measured when 5 parts by weight of the composition is uniformly dispersed and/or dissolved in 20 parts by weight pure water at 25°C. In particular the pH may be measured by manually mixing 5 g oral care composition with 20 mL water for 30 s, then immediately testing the pH with indicator or a pH meter.

### Calcium Ion Concentration

"Calcium ion concentration" for the purpose of the present invention is determined by using SevenExcellence™ device (Mettler Toledo Ltd, Switzerland) fitted with a Ca ion-selective electrode (ISE, perfectION™, Mettler Toledo Ltd, Switzerland). The sample to be analysed is prepared by adding an amount of raw material into deionized water. The Ca ISE is calibrated by addition of aliquots of calcium standard solution (Mettler Toledo Ltd) into deionized water prior to measurement. Data is recorded automatically every 5 seconds and five minutes are required for stable reading in the sample.

### Solubility

"Soluble" and "insoluble" for the purpose of the present invention means the solubility of a source (e.g., like calcium salts) in water at 25°C and atmospheric pressure. "Soluble" means a source that dissolves in water to give a solution with a concentration of at least 0.1 moles per litre. "Insoluble" means a source that dissolves in water to give a solution with a concentration of less than 0.001 moles per litre. "Slightly soluble", therefore, is defined to mean a source that dissolves in water to give a solution with a concentration of greater than 0.001 moles per litre and less than 0.1 moles per litre.

### Substantially Free

"Substantially free of" for the purpose of the present invention means less than 1.5%, and preferably less than 1.0%, and more preferably less than 0.75% and more preferably still less than 0.5%, and even more preferably less than 0.1% and most preferably from 0.0 to 0.01% by weight, based on total weight of the oral care composition, including all ranges subsumed therein.

### Anhydrous Composition

"Anhydrous composition" for the purpose of the present invention means the water content of the composition is less than 3%, preferably from 0.0 to 1.5% by total weight of the oral care composition.

### Dual-Phase

"Dual-Phase" for the purpose of the present invention means a composition having two independent phases which are physically separate.

### Viscosity

Viscosity of a tooth paste is the value taken at room temperature (25°C) with a Brookfield Viscometer, Spindle No.4 and at a speed of 5 rpm. Values are quoted in centipoises (cP=mPa.s) unless otherwise specified.

### Remineralisation

"Remineralisation" for the purpose of the present invention means *in situ* (i.e. in the oral cavity) generation of calcium phosphate on teeth (including layers on teeth from 10 nm to 40 microns, and preferably from 75 nm to 20 microns, and most preferably, from 150 nm to 10 microns thick including all ranges subsumed therein) to reduce the likelihood of tooth sensitivity, tooth decay, regenerate enamel and/or improve the appearance of teeth by whitening through the generation of such new calcium phosphate.

### Miscellaneous

Except in the examples, or where otherwise explicitly indicated, all numbers in this description indicating amounts of material or conditions of reaction, physical properties of materials and/or use may optionally be understood as modified by the word "about".

All amounts are by weight of the final oral care composition, unless otherwise specified.

It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

### Summary of the Invention

In a first aspect, the present invention is directed to an oral care composition comprising:
a) from 0.01 to 50% by weight of a calcium source;
b) a water soluble phosphate source;
c) a water soluble silicate source; and
d) from 0.01 to 10% by weight of an amino acid, or its physiologically acceptable salt, or a mixture thereof;
wherein the calcium source is calcium hydroxide, calcium oxide, calcium glycerophosphate, calcium lactate, calcium sulfate, calcium salts of citric acid, calcium chloride, calcium nitrate, calcium acetate, calcium gluconate, calcium formate, calcium malate, calcium propionate, calcium butyrate, calcium bicarbonate, monocalcium phosphate anhydrous, dicalcium phosphate anhydrous, tricalcium phosphate, octacalcium phosphate, calcium silicate, calcium carboxymethyl cellulose, calcium alginate, calcium carbonate or mixtures thereof;
wherein the phosphate source is trisodium phosphate, monosodium dihydrogen phosphate, disodium hydrogen phosphate, ammonium phosphate, diammonium hydrogen phosphate, ammonium dihydrogen phosphate, tripotassium phosphate, monopotassium dihydrogen phosphate, dipotassium hydrogen phosphate or a mixture thereof;
wherein the silicate source is sodium silicate, potassium silicate, tetraethylorthosilicate, tetraethylsilicate or mixtures thereof; and
wherein the amino acid is selected from acidic amino acid, neutral amino acid or a mixture thereof.

In a second aspect, the present invention is directed to a packaged oral care product comprising the oral care composition of the first aspect of this invention.

In a third aspect, the present invention is directed to a method for remineralising and/or whitening teeth of an individual comprising the step of applying the oral care composition of any embodiment of the first aspect to at least one surface of the teeth of the individual.

All other aspects of the present invention will more readily become apparent upon considering the detailed description and examples which follow.

### Brief Description of the Drawings

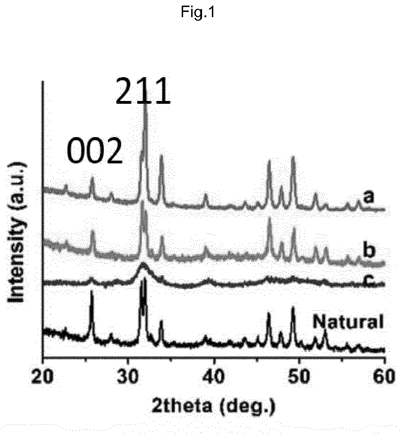

Figure 1 shows the X-ray diffraction pattern of the enamel surfaces. Lines a, b, and c are referred to samples comprising no PAA, 200 µg/mL PAA, and 350 µg/mL PAA respectively. The Line "natural" is referred to eroded native enamel surface.

### Detailed Description

Now it has been found that an oral care composition comprising a calcium source, a water soluble phosphate source, a water soluble silicate source and an amino acid has excellent tooth remineralisation efficacy, particularly such a composition can induce the formation of smaller HAP crystallites to form a dense remineralisation layer on tooth surfaces, which result in an enhancement of the binding affinity of the new remineralisation layer to tooth surfaces. Moreover, such an oral care composition comprising additional polyacrylic acid can even regulate the aggregation and crystallisation of the HAP crystallites formed on tooth surfaces into ordered structures which have the morphology and orientation similar to those of native enamel.

The calcium source suitable for use in this invention is limited only to the extent that the same may be used in the mouth. Preferably, the calcium source is capable of reacting with phosphate ions to produce a calcium phosphate *in situ. In situ,* as used herein, means during and/or after application of the oral care composition to the oral cavity. The calcium source dissolves in water to give a calcium ion concentration of at least 0.0005 moles per liter at room temperature and atmospheric pressure. Illustrative yet non-limiting examples of the types of calcium source that may be used in this invention include, for example, calcium hydroxide, calcium oxide, calcium glycerophosphate, calcium lactate, calcium sulfate, calcium salts of citric acid, calcium chloride, calcium nitrate, calcium acetate, calcium gluconate, calcium formate, calcium malate, calcium propionate, calcium butyrate, calcium bicarbonate, monocalcium phosphate anhydrous, dicalcium phosphate anhydrous, tricalcium phosphate, octacalcium phosphate, calcium silicate, calcium carboxymethyl cellulose, calcium alginate, calcium carbonate, mixtures thereof or the like.

Preferably, the calcium source comprises calcium chloride, calcium nitrate, calcium glycerophosphate, calcium lactate, calcium acetate, calcium gluconate, calcium carbonate, calcium silicate or mixtures thereof. Most preferably the calcium source comprises calcium chloride, calcium acetate, calcium gluconate, calcium glycerophosphate, calcium silicate or mixtures thereof.

The oral care composition comprises from 0.01 to 50% by weight of the calcium source, preferably from 0.1 to 30%, and more preferably from 1 to 20%, based on the total weight of the oral care composition and including all ranges subsumed therein.

The water soluble phosphate source that may be used in this invention is limited only to the extent that the same may be used in a composition suitable for use in the mouth. The phosphate source dissolves in water to give a phosphate ion concentration of at least 0.005 moles per liter at room temperature and atmospheric pressure. The phosphate ion concentration can be measured, for example, by ICP-OES (Inductively Coupled Plasma Optical Emission Spectroscopy).

Illustrative examples of the types of phosphate source suitable for use in this invention include trisodium phosphate, monosodium dihydrogen phosphate, disodium hydrogen phosphate, ammonium phosphate, diammonium hydrogen phosphate, ammonium dihydrogen phosphate, tripotassium phosphate, monopotassium dihydrogen phosphate, dipotassium hydrogen phosphate, mixtures thereof or the like.

The phosphate source typically makes up from 0.05 to 50%, and more preferably, from 2 to 40%, and most preferably, from 5 to 35% by weight of the oral care composition, based on total weight of the oral care composition and including all ranges subsumed therein.

Preferably, the phosphate source results in an oral care composition having a pH from 5.0 to 10.0, more preferably from 5.5 to 8.0, and most preferably from 6.0 to 7.5.

The oral care composition preferably comprises the calcium source and the phosphate source in a weight ratio from 10:1 to 1:30, preferably from 5:1 to 1:20, most preferably from 3:1 to 1:15.

The present inventors have surprisingly found that certain additives like silicate ions and amino acids can enhance the binding affinity of a new remineralisation layer to tooth surfaces. Without wishing to be bound by theory, the present inventors believe that the calcium source and the phosphate source of this invention allow for the formation of HAP crystallites on tooth surfaces and that the silicate ions and amino acids affect the HAP nucleation kinetics which induce the formation of smaller HAP crystallites and a dense remineralisation layer on tooth surfaces.

The silicate source suitable for use in this invention is limited only to the extent that the same may be used in the mouth. Illustrative yet non-limiting examples of the types of silicate source suitable for use in this invention include, for example, sodium silicate, potassium silicate, tetraethyl orthosilicate, tetraethylsilicate, mixtures thereof or the like. In a preferred embodiment, the silicate source is sodium silicate.

Typically, the oral care composition of the present invention comprises from 0.005 to 5% by weight of the silicate source, more preferably from 0.01 to 3%, most preferably from 0.03 to 1%, based on the total weight of the oral care composition and including all ranges subsumed therein.

The amino acids may further help HAP crystallites to form ordered structures through the interaction of the carboxylic acid groups of the amino acid and the HAP crystallites when the oral care composition comprises the amino acids in an amount of at least 1% by weight of the composition. However, such a high concentration of amino acids in oral care compositions is not favourable which limits its application. To regulate the aggregation and crystallisation of the HAP crystallites, the composition of the present invention may further comprise polyacrylic acid.

The amino acid suitable for use in this invention is selected from an acidic amino acid, neutral amino acid or a mixture thereof. Suitable amino acids include, for example, glutamic acid, glycine, aspartic acid, alanine, leucine or a mixture thereof. The carboxylic acid groups of the amino acid may be the key motif required for interaction with the HAP crystallites. Thus it is preferred that the amino acid has at least 2 carboxylic acid groups. Preferably the amino acid is acidic amino acid especially glutamic acid, aspartic acid or a mixture thereof. More preferably, the amino acid is glutamic acid.

Suitable physiologically acceptable salts of the amino acid include those in which the couterion is selected from the group consisting of Na⁺, K⁺, NH₄⁺, Mg²⁺, Al³⁺, Zn²⁺ and mixtures thereof. More preferably are salts with monovalent ions as these tend to be very water soluble. Thus most preferably the couterion is selected from the group consisting of Na⁺, K⁺, NH₄⁺, and mixtures thereof.

The oral care composition of the present invention comprises from 0.01 to 10% by weight of the amino acid, or its physiologically acceptable salt, or a mixture thereof, preferably from 0.05 to 5%, more preferably from 0.1 to 3%.

It should be noted that where amino acid is mentioned in the present disclosure, this also includes the corresponding physiologically acceptable salts thereof, also where not explicitly stated.

The oral care composition of the present invention may further comprise polyacrylic acid, or its physiologically acceptable salt, or a mixture thereof. In particular, polyacrylic acid with a molar mass ranging from 1000 to 10,000 g mol⁻¹ is preferred, more preferably the molar mass ranges from 1800 to 5100 g mol⁻¹. Where molar mass is stated, these do not include the mass of any couterions or water of hydration.

Typically, the oral care composition of the present invention comprises from 0.001 to 1% by weight of the polyacrylic acid, more preferably from 0.005 to 0.5%, most preferably from 0.01 to 0.1%, based on the total weight of the oral care composition and including all ranges subsumed therein.

The composition of the present invention is an oral care composition and typically comprises physiologically acceptable carrier. The carrier preferably comprises at least surfactant, thickener, humectant or a combination thereof.

Preferably the oral care composition comprises a surfactant. Preferably the composition comprises at least 0.01% surfactant by weight of the composition, more preferably at least 0.1% and most preferably from 0.5 to 7%. Suitable surfactants include anionic surfactants, such as the sodium, magnesium, ammonium or ethanolamine salts of C₈ to C₁₈ alkyl sulphates (for example sodium lauryl sulphate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used. More preferably the surfactant comprises or is anionic surfactant. The preferred anionic surfactants are sodium lauryl sulphate and/or sodium dodecylbenzene sulfonate. Most preferably the surfactant is sodium lauryl sulphate, sodium coco sulfate, cocamidopropyl betaine, sodium methyl cocoyl taurate or mixtures thereof.

Thickener may also be used in this invention and is limited only to the extent that the same may be added to a composition suitable for use in the mouth. Illustrative examples of the types of thickeners that may be used in this invention include, sodium carboxymethyl cellulose (SCMC), hydroxyl ethyl cellulose, methyl cellulose, ethyl cellulose, gum tragacanth, gum arabic, gum karaya, sodium alginate, carrageenan, guar, xanthan gum, Irish moss, starch, modified starch, silica based thickeners including silica aerogels, magnesium aluminum silicate (e.g., Veegum), Carbomers (cross-linked acrylates) and mixtures thereof.

Typically, xanthan gum and/or sodium carboxymethyl cellulose and/or a Carbomer is/are preferred. When a Carbomer is employed, those having a weight-average molecular weight of at least 700,000 are desired, and preferably, those having a molecular weight of at least 1,200,000, and most preferably, those having a molecular weight of at least about 2,500,000 are desired. Mixtures of Carbomers may also be used herein.

In an especially preferred embodiment, the Carbomer is Synthalen PNC, Synthalen KP or a mixture thereof. It has been described as a high molecular weight and cross-linked polyacrylic acid and identified via CAS number 9063-87-0. These types of materials are available commercially from suppliers like Sigma.

In another especially preferred embodiment, the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4. The same is available from suppliers like Alfa Chem.

In another especially preferred embodiment, the thickener is xanthan gum.

Thickener typically makes up from 0.01 to about 10%, more preferably from 0.1 to 9%, and most preferably, from 0.1 to 2% by weight of the oral care composition, based on total weight of the composition and including all ranges subsumed therein.

When the oral care composition of this invention is a toothpaste or gel, the same typically has a viscosity from about 30,000 to 180,000 centipoise, and preferably, from 60,000 to 170,000 centipoise, and most preferably, from 65,000 to 165,000 centipoise.

Suitable humectants are preferably used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. Glycerin, polyethylene glycol, sorbitol or mixtures thereof are the preferred humectants.

The humectant may be present in the range of from 10 to 90% by weight of the oral care composition. More preferably, the carrier humectant makes up from 25 to 80%, and most preferably, from 45 to 70% by weight of the composition, based on total weight of the composition and including all ranges subsumed therein.

The oral care composition of the present invention may contain a variety of other ingredients which are common in the art to enhance physical properties and performance. These ingredients include antimicrobial agents, anti-inflammatory agents, anti-caries agents, plaque buffers, fluoride sources, vitamins, plant extracts, desensitizing agents, anti-calculus agents, biomolecules, flavors, proteinaceous materials, preservatives, opacifying agents, coloring agents, pH-adjusting agents, sweetening agents, particulate abrasive materials, polymeric compounds, buffers and salts to buffer the pH and ionic strength of the compositions, and mixtures thereof. Such ingredients typically and collectively make-up less than 20% by weight of the composition, and preferably, from 0.0 to 15% by weight, and most preferably, from 0.01 to 12% by weight of the composition, including all ranges subsumed therein.

The oral care composition of this invention can be used in a method of remineralising and/or whitening of teeth of an individual comprising applying the composition to at least one surface of the teeth of an individual. The oral care composition of this invention may additionally or alternatively be for use as a medicament and/or used in the manufacture of a medicament for providing an oral care benefit as described herein, such as for remineralising of the teeth of an individual. Alternatively and preferably, the use is non-therapeutic.

Preferably, the oral care composition is substantially free of water to prevent the premature reaction between the calcium source and the phosphate source.

In a preferred embodiment, the oral care composition is a monophase anhydrous composition.

In another preferred embodiment, the oral care composition is a dual-phase composition comprising a calcium phase and a water soluble phosphate phase, wherein the calcium source is present in the calcium phase, the phosphate source, the water soluble silicate source, and the amino acid are present in the phosphate phase. When PAA is included into the composition, it is present in the phosphate phase. The two phases are physically separate from one another by being in independent phases. The delivery of the two independent phases to the teeth may be simultaneous or sequential. In a preferred embodiment, the phases are delivered simultaneously.

Typically, the dual-phase composition is delivered by a dual-tube having a first compartment for calcium phase and a second compartment for phosphate phase, which allows for co-extrusion of the two phases.

In a preferred embodiment, such a dual-tube has one of the compartments surrounding the other. In such embodiments, one phase is present as a sheath, surrounding the other phase in the core. In an especially preferred embodiment, the core is the calcium phase and the sheath is the phosphate phase.

In another preferred embodiment, such a dual-tube has the two compartments side by side within the same tube. In such embodiments, the two phases are extruded from the tube as one, such extrusion being termed "contact extrusion". A pump head may be used in such a dual-tube for squeezing the two phases from the tube as one.

The dual-phase oral care composition may be a gel composition that comprises two independent gel phases, the first is the calcium phase and the second is the phosphate phase. The means of delivery may involve a cotton rod, or a tray, onto which the calcium phase and the phosphate phase are applied, prior to the tray being placed in contact with the teeth.

Typically the composition will be packaged. In tooth paste or gel form, the composition may be packaged in a conventional plastic laminate, metal tube or a single compartment dispenser. The same may be applied to dental surfaces by any physical means, such as a toothbrush, fingertip or by an applicator directly to the sensitive area.

The composition can be effective even when used in an individual's daily oral hygiene routine. For example, the composition may be brushed onto the teeth. The composition may, for example, be contacted with the teeth for a time period of one second to 20 hours. More preferably from 1 s to 10 hours, more preferably still from 10 s to 1 hour and most preferably from 30 s to 5 minutes. The composition may be used daily, for example for use by an individual once, twice or three times per day. When the oral care composition is a dual-phase composition, the two phases of the composition are mixed during application. The mixed phases are typically left on the teeth for from 3 minutes to 10 hours, more preferably from 3 minutes to 8 hours. The application may be carried out daily.

The following examples are provided to facilitate an understanding of the present invention. The examples are not provided to limit the scope of the claims.

### Examples

### Example 1

This example demonstrates the effect of silicate source on enamel remineralisation.

### Preparation of Remineralisation Solutions

### Materials

All of the chemicals used in this study were purchased from Aladdin Reagent (Shanghai, China) with analytical grade unless specifically mentioned. Triple distilled water was used and all solutions were filtered through 0.22 µm Millipore films prior to use.

### Remineralisation Solution

The remineralisation solution was prepared by mixing equal volumes of a calcium containing solution (Ca solution) and a phosphate containing neutralised buffered solution (P solution). The calcium solution was prepared by dissolving a calculated amount of CaCl₂ and MgCl₂•6H₂O. The phosphate containing solution was prepared by using Na₂HPO₄, KCI, NaCl, Na₂SO₄, NaHCO₃, Glutamic acid (Glu), polyacrylic acid (PAA) and tris(hydroxymethyl) aminomethane (Tris, from Sigma-Aldrich, St. Louis, MO, USA). Sodium silicate (Na₂SiO_{3•}9H₂O) stock solution was introduced to the phosphate solution before mixing. After mixing, the final concentration of each species in the remineralisation solution was listed in Table 1. The concentrations of Glu, silicate ion and PAA were variants which were represented by X,Y and Z respectively. The concentrations of Glu, silicate ion and PAA in different samples were listed in Table 2.

**TABLE 1**

| **Ions** | **Na⁺** | **Mg²⁺** | **K⁺** | **Cl⁻** | **SO₄²⁻** | **Ca²⁺** | **HPO₄²⁻** | **HCO₃⁻** | **Tris** | **Glu** | **SiO₃²⁻** | **PAA** |
|---|---|---|---|---|---|---|---|---|---|---|---|---|
| Concentration/ mM | 142.0 | 1.5 | 17.0 | 147.8 | 0.5 | 0.9 | 7.2 | 4.2 | 115 | X | Y | Z |

**TABLE 2**

| **Ingredients** | **Samples** | | | | |
|---|---|---|---|---|---|
| | **1** | **2** | **3** | **4** | **5** |
| Silicate ions(mM) | -- | 5 | 10 | 15 | 15 |
| Glu(mM) | 10 | 10 | 10 | 10 | -- |

### Methods

Enamel slabs obtained from selected bovine incisor teeth were stored in 0.1% thymol solution at 4°C. The enamel slabs were fixed on acrylic rods with sticky wax and polished. The area of a typical enamel window was 5x5 mm². The well-polished enamel slabs were eroded by 37% phosphoric acid for 10s under ultrasonic treatment and then rinsed with de-ionised (DI) water and ethyl alcohol respectively before gently dried with paper.

Each enamel slab was placed into a glass vial filled with 10 mL remineralisation solution. The glass vial was capped with a plastic lid to prevent evaporation of the solution and stored in an incubator at 37.0°C to mimic physiological condition. The remineralisation solution was replaced every 12 hours. The enamel slabs were taken out from the remineralisation solution after 6 days, and then put into distilled water and ultrasonic sonication for 5 mins. After drying, the enamel slabs were characterised with scanning electron microscope (SEM, HITACHI S4800) and X-ray diffraction (XRD, Rigaku D/MAX-2550pc, Cu Kα=1.54Å).

### Results

The eroded tooth enamel slabs showed needle-like apatite crystallites. After 6 days of treatment with different remineralisation solutions, it can be seen from the SEM images that a new layer was formed on all the enamel slabs. Irregular mineral particles were formed on enamel surfaces for Sample 1 without silicate ions. For Sample 2 comprising 5 mM of silicate, it showed the deposition of granular-like particles on enamel surfaces. But for Samples 3 to 5 comprising higher concentration of silicate ions, it showed the formation of a dense layer of small crystallites.

To evaluate the binding affinity of the newly formed layer to enamel surfaces, the enamel slabs after 6 days of treatment with remineralisation solutions were further treated by ultrasonic sonication for 30 mins. For Sample 1 without silicate ions, the newly formed layer was completely lost, and the needle-like crystallites of the eroded enamel slabs were exposed. Similar result was observed for Sample 5 comprising no Glu, after ultrasonic sonication, the needle-like crystallites of the eroded enamel slabs were also exposed. But for Sample 4, the newly formed layer was well remained, indicating enhanced binding affinity of the newly formed layer to enamel surfaces.

### Example 2

This example demonstrates the effect of polyacrylic acid on enamel remineralisation.

### Preparation of Remineralisation Solutions

The same protocol was used to prepare the remineralisation solution as described in Example 1. After mixing, the final concentration of each species in the remineralisation solution was listed in Table 1. The concentration of Glu, silicate ion and PAA in different samples were listed in Table 3.

**TABLE 3**

| **Ingredient** | **Samples** | | |
|---|---|---|---|
| | **6** | **7** | **8** |
| Silicate ions(mM) | 15 | 15 | 15 |
| Glu(mM) | 20 | 20 | 20 |
| PAA(µg/mL) | -- | 200 | 350 |

### Methods

The same protocol was used to evaluate the enamel remineralisation as described in Example 1.

### Results

After 6 days of treatment with different remineralisation solutions, the SEM images showed a new layer was formed on all the enamel slabs. For Sample 6, there was no observation of ordered structure formed on enamel surfaces in the absence of PAA. For Samples 7 and 8 comprising PAA, it showed the formation of ordered sheet-like crystallites. Moreover, with the increase of PAA concentration, the newly formed layer became smoother and denser.

The XRD patterns of the newly formed layer on enamel surfaces were shown in Fig.1, which further confirmed that the newly formed layer on enamel surfaces was HAP. The unsplit diffraction peak around 2θ of 32° indicated the crystallinity of newly formed HAP.

The ratio of different XRD peak intensities can be used to estimate the specific orientation of crystals at the macroscopic level. The ratio (R) of 2θ=25.8° (002) and 2θ=32° (211) peak intensities of HAP can indicate the preference of crystals to be aligned along the crystallographic c-axis. The specific R of native enamel used in this experiment was 0.83 (Fig. 1, line "natural). In the absence of PAA, the R of the new layer formed was only 0.31 (Fig.1, line a). For Samples 7 and 8, the R increased to 0.50 and 0.60 respectively (Fig.1, line b and line c), indicating the orientation of the HAP crystallites was closer to that of the native enamel.

### Example 3

This example demonstrates the effect of glutamic acid, silicate source and polyacrylic acid on enamel remineralisation.

### Preparation of Remineralisation Solutions

The same protocol was used to prepare the remineralisation solution as described in Example 1. After mixing, the final concentration of each species in the remineralisation solution was listed in Table 1. The concentration of Glu, silicate ion and PAA in different samples were listed in Table 4.

**TABLE 4**

| **Ingredient** | **Samples** | | | | |
|---|---|---|---|---|---|
| | **7** | **9** | **10** | **11** | **12** |
| Silicate ions(mM) | 15 | 15 | 15 | 5 | 10 |
| Glu(mM) | 20 | 15 | 10 | 20 | 20 |
| PAA(µg/mL) | 200 | 200 | 200 | 200 | 200 |

### Methods

The same protocol was used to evaluate the enamel remineralisation as described in Example 1.

### Results

After 6 days of treatment with different remineralisation solutions, the SEM images showed a new layer was formed on all the enamel slabs. For Sample 10 comprising 10 mM of Glu, there was no observation of ordered structure formed on enamel surfaces. For Samples 7 and 9 comprising higher concentration of Glu, it showed the formation of ordered sheet-like crystallites.

For Samples 7, 9 and 10 comprising higher concentration of silicate ions, the newly formed layer was a closely packed dense layer of small and fine crystallites.

## Claims

1. An oral care composition comprising:
a) from 0.01 to 50% by weight of a calcium source;
b) a water soluble phosphate source;
c) a water soluble silicate source; and
d) from 0.01 to 10% by weight of an amino acid, or its physiologically acceptable salt, or a mixture thereof;
wherein the calcium source is calcium hydroxide, calcium oxide, calcium glycerophosphate, calcium lactate, calcium sulfate, calcium salts of citric acid, calcium chloride, calcium nitrate, calcium acetate, calcium gluconate, calcium formate, calcium malate, calcium propionate, calcium butyrate, calcium bicarbonate, monocalcium phosphate anhydrous, dicalcium phosphate anhydrous, tricalcium phosphate, octacalcium phosphate, calcium silicate, calcium carboxymethyl cellulose, calcium alginate, calcium carbonate or mixtures thereof;
wherein the phosphate source is trisodium phosphate, monosodium dihydrogen phosphate, disodium hydrogen phosphate, ammonium phosphate, diammonium hydrogen phosphate, ammonium dihydrogen phosphate, tripotassium phosphate, monopotassium dihydrogen phosphate, dipotassium hydrogen phosphate or a mixture thereof;
wherein the silicate source is sodium silicate, potassium silicate, tetraethylorthosilicate, tetraethylsilicate or mixtures thereof; and
wherein the amino acid is selected from acidic amino acid, neutral amino acid or a mixture thereof.

2. The oral care composition according to claim 1, wherein the calcium source is calcium chloride, calcium nitrate, calcium glycerophosphate, calcium lactate, calcium acetate, calcium gluconate, calcium carbonate, calcium silicate or mixtures thereof, preferably calcium chloride, calcium acetate, calcium gluconate, calcium glycerophosphate, calcium silicate or mixtures thereof.

3. The oral care composition according claim 1 or claim 2, wherein the composition comprises the calcium source in an amount of from 0.1 to 30% by weight of the composition, preferably from 1 to 20%.

4. The oral care composition according to any of the preceding claims, wherein the composition comprises the phosphate source in an amount of from 0.05 to 50% by weight of the composition, preferably from 2 to 40%.

5. The oral care composition according to any of the preceding claims, wherein the composition comprises the calcium source and the phosphate source in a weight ratio from 10:1 to 1:30, preferably from 5:1 to 1:20.

6. The oral care composition according to any of the preceding claims, wherein the composition comprises the silicate source in an amount of from 0.005 to 5% by weight of the composition, preferably from 0.01 to 3%.

7. The oral care composition according to any of the preceding claims, wherein the amino acid is glutamic acid, glycine, aspartic acid, asparagine, alanine, leucine or a mixture thereof, preferably glutamic acid, aspartic acid or a mixture thereof.

8. The oral care composition according to claim 7, wherein the amino acid is glutamic acid.

9. The oral care composition according to any of the preceding claims, wherein the composition comprises the amino acid in an amount of from 0.05 to 5% by weight of the composition, preferably from 0.1 to 3%.

10. The oral care composition according to any of the preceding claims, wherein the composition further comprises polyacrylic acid, or its physiologically acceptable salt, or a mixture thereof.

11. The oral care composition according to any of the preceding claims, wherein the polyacrylic acid has a molar mass ranging from 1000 to 10000 g mol⁻¹, preferably from 1800 to 5100 g mol⁻¹.

12. The oral care composition according to claim 10 or claim 11, wherein the composition comprises the polyacrylic acid in an amount of from 0.001 to 1% by weight of the composition, preferably from 0.005 to 0.5%.

13. The oral care composition according to any of the preceding claims, wherein the oral care composition is a monophase anhydrous composition.

14. The oral care composition according to any one of claims 1 to 12, wherein the composition is a dual-phase composition comprising a calcium phase and a phosphate phase, wherein the calcium source is present in the calcium phase, and the phosphate source, the water soluble silicate source and the amino acid are present in the phosphate source.

15. A method for remineralising the teeth of an individual comprising the step of applying the composition as claimed in any of the preceding claims to at least one surface of the teeth of the individual.

## Patentansprüche

1. Mundpflegezusammensetzung, umfassend:
a) 0,01 bis 50 Gewichts-% einer Calciumquelle;
b) eine wasserlösliche Phosphatquelle;
c) eine wasserlösliche Silicatquelle; und
d) 0,01 bis 10 Gewichts-% einer Aminosäure oder ihres physiologisch verträglichen Salzes oder einer Mischung davon;
wobei die Calciumquelle Calciumhydroxid, Calciumoxid, Calciumglycerophosphat, Calciumlactat, Calciumsulfat, Calciumsalze der Citronensäure, Calciumchlorid, Calciumnitrat, Calciumacetat, Calciumgluconat, Calciumformiat, Calciummalat, Calciumpropionat, Calciumbutyrat, Calciumbicarbonat, Monocalciumphosphat wasserfrei, Dicalciumphosphat wasserfrei, Tricalciumphosphat, Octacalciumphosphat, Calciumsilicat, Calciumcarboxymethylcellulose, Calciumalginat, Calciumcarbonat oder Mischungen davon ist;
wobei die Phosphatquelle Trinatriumphosphat, Mononatriumdihydrogenphosphat, Dinatriumhydrogenphosphat, Ammoniumphosphat, Diammoniumhydrogenphosphat, Ammoniumdihydrogenphosphat, Trikaliumphosphat, Monokaliumdihydrogenphosphat, Dikaliumhydrogenphosphat oder eine Mischung davon ist;
wobei die Silicatquelle Natriumsilicat, Kaliumsilicat, Tetraethylorthosilicat, Tetraethylsilicat oder Mischungen davon ist; und
wobei die Aminosäure ausgewählt ist aus saurer Aminosäure, neutraler Aminosäure oder einer Mischung davon.

2. Mundpflegezusammensetzung nach Anspruch 1, wobei die Calciumquelle Calciumchlorid, Calciumnitrat, Calciumglycerophosphat, Calciumlactat, Calciumacetat, Calciumgluconat, Calciumcarbonat, Calciumsilicat oder Mischungen davon ist, bevorzugt Calciumchlorid, Calciumacetat, Calciumgluconat, Calciumglycerophosphat, Calciumsilicat oder Mischungen davon.

3. Mundpflegezusammensetzung nach Anspruch 1 oder Anspruch 2, wobei die Zusammensetzung die Calciumquelle in einer Menge von 0,1 bis 30 Gewichts-% der Zusammensetzung, bevorzugt 1 bis 20 %, umfasst.

4. Mundpflegezusammensetzung nach irgendeinem der vorhergehenden Ansprüche, wobei die Zusammensetzung die Phosphatquelle in einer Menge von 0,05 bis 50 Gewichts-% der Zusammensetzung, bevorzugt 2 bis 40%, umfasst.

5. Mundpflegezusammensetzung nach irgendeinem der vorhergehenden Ansprüche, wobei die Zusammensetzung die Calciumquelle und die Phosphatquelle in einem Gewichtsverhältnis von 10:1 bis 1:30, bevorzugt von 5:1 bis 1:20, umfasst.

6. Mundpflegezusammensetzung nach irgendeinem der vorhergehenden Ansprüche, wobei die Zusammensetzung die Silicatquelle in einer Menge von 0,005 bis 5 Gewichts-% der Zusammensetzung, bevorzugt von 0,01 bis 3 %, umfasst.

7. Mundpflegezusammensetzung nach irgendeinem der vorhergehenden Ansprüche, wobei die Aminosäure Glutaminsäure, Glycin, Asparaginsäure, Asparagin, Alanin, Leucin oder eine Mischung davon, bevorzugt Glutaminsäure, Asparaginsäure oder eine Mischung davon, ist.

8. Mundpflegezusammensetzung nach Anspruch 7, wobei die Aminosäure Glutaminsäure ist.

9. Mundpflegezusammensetzung nach irgendeinem der vorhergehenden Ansprüche, wobei die Zusammensetzung die Aminosäure in einer Menge von 0,05 bis 5 Gewichts-% der Zusammensetzung, bevorzugt von 0,1 bis 3%, umfasst.

10. Mundpflegezusammensetzung nach irgendeinem der vorhergehenden Ansprüche, wobei die Zusammensetzung ferner Polyacrylsäure oder ihr physiologisch verträgliches Salz oder eine Mischung davon umfasst.

11. Mundpflegezusammensetzung nach irgendeinem der vorhergehenden Ansprüche, wobei die Polyacrylsäure eine Molmasse im Bereich von 1000 bis 10000 g mol⁻¹, bevorzugt von 1800 bis 5100 g mol⁻¹, aufweist.

12. Mundpflegezusammensetzung nach Anspruch 10 oder Anspruch 1 1, wobei die Zusammensetzung die Polyacrylsäure in einer Menge von 0,001 bis 1 Gewichts-% der Zusammensetzung, bevorzugt von 0,005 bis 0,5%, umfasst.

13. Mundpflegezusammensetzung nach irgendeinem der vorhergehenden Ansprüche, wobei die Mundpflegezusammensetzung eine einphasige wasserfreie Zusammensetzung ist.

14. Mundpflegezusammensetzung nach irgendeinem der Ansprüche 1 bis 12, wobei die Zusammensetzung eine zweiphasige Zusammensetzung ist, die eine Calciumphase und eine Phosphatphase umfasst, wobei die Calciumquelle in der Calciumphase vorhanden ist und die Phosphatquelle, die wasserlösliche Silicatquelle und Aminosäure in der Phosphatquelle vorhanden sind.

15. Verfahren zum Remineralisieren der Zähne eines Individuums, umfassend den Schritt des Aufbringens der Zusammensetzung, wie in irgendeinem der vorhergehenden Ansprüche beansprucht, auf mindestens eine Oberfläche der Zähne des Individuums.

## Revendications

1. Composition pour le soin oral comprenant :
a) de 0,01 à 50 % en masse d'une source de calcium ;
b) une source de phosphate soluble dans l'eau ;
c) une source de silicate soluble dans l'eau ; et
d) de 0,01 à 10 % en masse d'un acide aminé, ou son sel physiologiquement acceptable, ou un mélange de ceux-ci ;
où la source de calcium est l'hydroxyde de calcium, oxyde de calcium, glycérophosphate de calcium, lactate de calcium, sulfate de calcium, sels de calcium d'acide citrique, chlorure de calcium, nitrate de calcium, acétate de calcium, gluconate de calcium, formate de calcium, malate de calcium, propionate de calcium, butyrate de calcium, bicarbonate de calcium, phosphate de monocalcium anhydre, phosphate de dicalcium anhydre, phosphate de tricalcium, phosphate d'octacalcium, silicate de calcium, calcium carboxyméthylcellulose, alginate de calcium, carbonate de calcium ou des mélanges de ceux-ci ;
où la source de phosphate est le phosphate de trisodium, dihydrogénophosphate de monosodium, hydrogénophosphate de disodium, phosphate d'ammonium, hydrogénophosphate de diammonium, dihydrogénophosphate d'ammonium, phosphate de tripotassium, dihydrogénophosphate de monopotassium, hydrogénophosphate de dipotassium ou un mélange de ceux-ci ;
où la source de silicate est le silicate de sodium, silicate de potassium, tétraéthylorthosilicate, tétraéthylsilicate ou des mélanges de ceux-ci ; et
où l'acide aminé est choisi parmi un acide aminé acide, acide aminé neutre ou un mélange de ceux-ci.

2. Composition pour le soin oral selon la revendication 1, dans laquelle la source de calcium est le chlorure de calcium, nitrate de calcium, glycérophosphate de calcium, lactate de calcium, acétate de calcium, gluconate de calcium, carbonate de calcium, silicate de calcium ou des mélanges de ceux-ci, de préférence chlorure de calcium, acétate de calcium, gluconate de calcium, glycérophosphate de calcium, silicate de calcium ou des mélanges de ceux-ci.

3. Composition pour le soin oral selon la revendication 1 ou revendication 2, dans laquelle la composition comprend la source de calcium dans une quantité de 0,1 à 30 % en masse de la composition, de préférence de 1 à 20 %.

4. Composition pour le soin oral selon l'une quelconque des revendications précédentes, dans laquelle la composition comprend la source de phosphate dans une quantité de 0,05 à 50 % en masse de la composition, de préférence de 2 à 40 %.

5. Composition pour le soin oral selon l'une quelconque des revendications précédentes, dans laquelle la composition comprend la source de calcium et la source de phosphate dans un rapport massique de 10:1 à 1:30, de préférence de 5:1 à 1:20.

6. Composition pour le soin oral selon l'une quelconque des revendications précédentes, dans laquelle la composition comprend la source de silicate dans une quantité de 0,005 à 5 % en masse de la composition, de préférence de 0,01 à 3 %.

7. Composition pour le soin oral selon l'une quelconque des revendications précédentes, dans laquelle l'acide aminé est l'acide glutamique, la glycine, l'acide aspartique, l'asparagine, l'alanine, la leucine ou un mélange de ceux-ci, de préférence l'acide glutamique, l'acide aspartique ou un mélange de ceux-ci.

8. Composition pour le soin oral selon la revendication 7, dans laquelle l'acide aminé est l'acide glutamique.

9. Composition pour le soin oral selon l'une quelconque des revendications précédentes, dans laquelle la composition comprend l'acide aminé dans une quantité de 0,05 à 5 % en masse de la composition, de préférence de 0,1 à 3 %.

10. Composition pour le soin oral selon l'une quelconque des revendications précédentes, dans laquelle la composition comprend de plus du poly(acide acrylique), ou son sel physiologiquement acceptable, ou un mélange de ceux-ci.

11. Composition pour le soin oral selon l'une quelconque des revendications précédentes, dans laquelle le poly(acide acrylique) présente une masse molaire de 1 000 à 10 000 g mol⁻¹, de préférence de 1 800 à 5 100 g mol⁻¹.

12. Composition pour le soin oral selon la revendication 10 ou revendication 11, dans laquelle la composition comprend le poly(acide acrylique) dans une quantité de 0,001 à 1 % en masse de la composition, de préférence de 0,005 à 0,5 %.

13. Composition pour le soin oral selon l'une quelconque des revendications précédentes, dans laquelle la composition pour le soin oral est une composition anhydre monophase.

14. Composition pour le soin oral selon l'une quelconque des revendications 1 à 12, dans laquelle la composition est une composition de phase double comprenant une phase de calcium et une phase de phosphate, dans laquelle la source de calcium est présente dans la phase de calcium, et la source de phosphate, la source de silicate soluble dans l'eau et l'acide aminé sont présents dans la source de phosphate.

15. Procédé pour la reminéralisation des dents d'un individu comprenant l'étape d'application de la composition selon l'une quelconque des revendications précédentes sur au moins une surface des dents de l'individu.
